# EUROPÄISCHE PATENTSCHRIFT

(11) **EP 4 659 783 B1**
(45) Veröffentlichungstag und Bekanntmachung des Hinweises auf die Patenterteilung: **19.08.2026**
(21) Anmeldenummer: 25180775.6
(22) Anmeldetag: 04.06.2025
(51) Int. Cl.: A61M 16/00, A61M 16/20

(54) **HUSTENGERÄT ZUM UNTERSTÜTZEN EINES PATIENTEN BEIM HUSTEN**
COUGHING DEVICE FOR ASSISTING A PATIENT TO COUGHING
DISPOSITIF POUR AIDER UN PATIENT À TOUX

(30) Priorität: 05.06.2024 DE 102024115615
(43) Veröffentlichungstag der Anmeldung: 10.12.2025
(73) Patentinhaber: Löwenstein Medical Technology S.A., 2557 Luxembourg (LU)
(72) Erfinder: Paasch, Lennart, 22761 Hamburg (DE); Schweikert, Kris, 25421 Pinneberg (DE); Schattner, Jan, 25421 Pinneberg (DE)
(74) Vertreter: Löwenstein Medical IP

(56) Entgegenhaltungen:
- EP-B1- 2 707 069
- EP-B1- 3 993 862
- DE-A1- 102018 008 619
- DE-A1- 102023 111 541
- DE-A1- 102023 115 183

## Beschreibung

### Technisches Gebiet

Die Erfindung betrifft ein Hustengerät zum Unterstützen eines Patienten beim Husten und ein Beatmungsgerät mit einem solchen Hustengerät.

### Stand der Technik

Ein Hustengerät, auch Insufflator-Exsufflator genannt, kann eingesetzt werden, um die Abfuhr von Sekreten aus den Atemwegen und/oder der Lunge eines Patienten mit beeinträchtigter Atemmuskulatur zu ermöglichen oder zu erleichtern. Zu diesem Zweck kann das Hustengerät die Atemwege und/oder die Lunge über eine geeignete Patientenschnittstelle abwechselnd mit einem Überdruck und einem Unterdruck beaufschlagen. Dadurch wird in kontrollierter Weise ein Hustenstoß oder eine Reihe von Hustenstößen verursacht. Die Effizienz der Sekretabfuhr bei einem einzelnen Hustenstoß hängt unter anderem vom Maximum des Volumenstroms bei der Exsufflation, auch Peak Cough Flow oder kurz PCF genannt, ab. Der PCF sollte möglichst hoch sein, um eine gute Sekretabfuhr zu ermöglichen. Gleichzeitig sollten übermäßige Druckspitzen bei der Exsufflation vermieden werden.

EP 2 707 069 B1 offenbart ein Hustengerät mit einer Drosseleinrichtung in Form einer Scheibe, die um 360 Grad zwischen verschiedenen Winkelpositionen mit verschiedenen Flussraten drehbar ist. Im Insufflationsmodus kann die Drosseleinrichtung in eine stationäre Position mit niedriger, mittlerer oder hoher Flussrate gestellt sein.

DE 10 2023 115183 A1 offenbart eine Atemtherapievorrichtung mit einer Drosseleinrichtung in Form eines Oszillationsventils zum Erzeugen eines sensorisch wahrnehmbaren Hinweissignals zum Signalisieren sich ändernder Atemgasparameter mittels einer entsprechenden Druck- und/oder Flussoszillation, beispielsweise bei der Insufflation.

### Offenbarung der Erfindung

Eine Aufgabe der Erfindung kann darin gesehen werden, ein Hustengerät bereitzustellen, das eine besonders effiziente Sekretabfuhr ermöglicht. Eine weitere Aufgabe der

Erfindung kann darin gesehen werden, ein Beatmungsgerät mit einem derart verbesserten Hustengerät bereitzustellen.

Diese Aufgaben werden durch die Gegenstände der unabhängigen Ansprüche gelöst. Vorteilhafte Ausführungsformen der Erfindung sind in den abhängigen Ansprüchen, der nachfolgenden Beschreibung und den beigefügten Figuren dargelegt.

Ein erster Aspekt der Erfindung betrifft ein Hustengerät nach Anspruch 1 zum Unterstützen eines Patienten beim Husten. Das Hustengerät umfasst: ein Gebläse mit einer Ansaugöffnung, einer Ausblasöffnung, einem Antriebsmotor und einem mit dem Antriebsmotor gekoppelten Rotor zum Fördern eines Gases von der Ansaugöffnung zur Ausblasöffnung abhängig von einer Motordrehzahl des Antriebsmotors; einen Druckanschluss; einen Patientenanschluss zum Anschließen einer Patientenschnittstelle; einen Verbindungskanal, der den Druckanschluss mit dem Patientenanschluss fluidisch verbindet; eine Koppeleinrichtung, die zwischen einer Insufflationsstellung und einer Exsufflationsstellung umschaltbar ist und ausgebildet ist, um den Druckanschluss in der Insufflationsstellung mit der Ausblasöffnung und in der Exsufflationsstellung mit der Ansaugöffnung fluidisch zu koppeln; eine Drosseleinrichtung, die zwischen einer Ausgangsstellung und einer Drosselstellung umschaltbar ist und ausgebildet ist, um den Verbindungskanal beim Schalten in die Drosselstellung zu verengen; einen Drucksensor, der ausgebildet ist, um einen am Patientenanschluss anliegenden Druck zu erfassen und ein den erfassten Druck anzeigendes Drucksignal zu erzeugen; eine Steuereinrichtung zum Steuern eines Betriebs des Hustengeräts. Die Steuereinrichtung ist ausgebildet, um folgendes Insufflationsverfahren auszuführen: Schalten der Koppeleinrichtung in die Insufflationsstellung; (anschließend oder als Reaktion auf das Schalten der Koppeleinrichtung in die Insufflationsstellung:) Empfangen des Drucksignals, wobei das Drucksignal einen am Patientenanschluss anliegenden Insufflationsdruck als den erfassten Druck anzeigt; Bestimmen einer Insufflationsdruckabweichung zwischen dem Insufflationsdruck und einem Insufflationssolldruck; Steuern des Antriebsmotors, um die Insufflationsdruckabweichung zu verringern; (beim Steuern des Antriebsmotors:) Schalten der Drosseleinrichtung in die Drosselstellung und Halten der Drosseleinrichtung in der Drosselstellung, um eine zusätzliche Erhöhung der Motordrehzahl zu bewirken.

Ein solches Hustengerät ermöglicht eine deutliche Steigerung des PCF, ohne dass ein leistungsstärkeres Gebläse oder eine schnellere Umschaltung von der Insufflations- in die Exsufflationsstellung erforderlich ist. Die Drosselung, speziell zum Ende der Insufflation hin, hat die Wirkung, dass ein dem Gebläse bei der Insufflation entgegenwirkender Strömungswiderstand zusätzlich erhöht wird. Aufgrund der Druckregelung erhöht sich die Motordrehzahl entsprechend. Dies ermöglicht es, in der anschließenden Exsufflation einen entsprechend erhöhten Volumenstrom bereitzustellen, da die erhöhte Motordrehzahl mindestens bis zum Zeitpunkt des Umschaltens in die Exsufflationsstellung beibehalten wird.

Das Hustengerät kann einen oder mehrere elektrische oder elektrisch steuerbare Aktoren zum Schalten der Koppeleinrichtung und/oder der Drosseleinrichtung umfassen. Ein solcher Aktor kann beispielsweise ein Elektromotor, ein Elektromagnet, ein Piezoelement oder eine Kombination von mindestens zwei dieser Beispiele sein.

Unter "Patientenschnittstelle" kann beispielsweise eine Mundmaske, eine Nasenmaske, ein Mundstück oder ein Tubus verstanden werden. Der Patientenanschluss kann beispielsweise über ein Schlauchsystem mit der Patientenschnittstelle fluidisch koppelbar sein.

Unter "Antriebsmotor" kann insbesondere ein Elektromotor verstanden werden.

Bei der Koppeleinrichtung und der Drosseleinrichtung kann es sich jeweils um ein elektrisch schaltbares Ventil handeln. Bei den Schaltstellungen der Koppeleinrichtung und/oder der Drosseleinrichtung kann es sich um diskrete Schaltstellungen handeln. Alternativ kann es sich bei den Schaltstellungen der Koppeleinrichtung und/oder der Drosseleinrichtung um Schaltstellungen eines kontinuierlich verstellbaren Ventils handeln.

Unter "Ausgangsstellung" kann eine Stellung der Drosseleinrichtung verstanden werden, bei der der Verbindungskanal entweder nicht verengt oder deutlich weniger als in der Drosselstellung verengt ist.

Unter "Drosselstellung" kann eine Stellung der Drosseleinrichtung verstanden werden, bei der der Verbindungskanal entweder vollständig verengt, d. h. gasdicht verschlossen ist oder teilweise (beispielsweise um mindestens 5 % und/oder höchstens 95 % relativ zur Ausgangsstellung) verengt ist, sodass weiterhin Gas durch den Verbindungskanal strömen kann. Je nach Ausführungsform kann eine Schaltgeschwindigkeit, mit der die Drosseleinrichtung von der Ausgangsstellung in die Drosselstellung bewegt wird, konstant sein oder entsprechend einem vorgegebenen Verlauf zeitlich variieren.

Unter "Verengen" kann allgemein eine Vergrößerung eines dem Gebläse im Betrieb des Hustengeräts entgegenwirkenden Strömungswiderstands verstanden werden. Wie bereits erwähnt, erhöht sich dabei die Motordrehzahl aufgrund der Druckregelung, um den durch die Verengung verursachten Druckabfall zu kompensieren. Anders ausgedrückt kann die Drosseleinrichtung allgemein als ein veränderbarer Strömungswiderstand aufgefasst werden.

Die Steuereinrichtung kann Hard- und/oder Softwarekomponenten umfassen. Beispielsweise kann die Steuereinrichtung einen Prozessor umfassen, der konfiguriert ist, um ein Computerprogramm zum Steuern des Betriebs des Hustengeräts auszuführen. Zusätzlich kann die Steuereinrichtung einen Speicher und/oder eine Datenkommunikationsschnittstelle zur drahtlosen und/oder drahtgebundenen Datenkommunikation mit Peripheriegeräten umfassen. Die Steuereinrichtung kann auch ausschließlich als Hardware, beispielsweise in Form eines ASIC- oder FPGA-Bausteins, implementiert sein.

Unter "Insufflationsdruck" kann ein Überdruck oder positiver Druck relativ zum jeweiligen Atmosphärendruck verstanden werden.

Ein zweiter Aspekt der Erfindung betrifft ein Beatmungsgerät zum invasiven und/oder nicht invasiven Beatmen eines Patienten. Das Beatmungsgerät umfasst ein Hustengerät, wie es vor- und nachstehend beschrieben wird. Im Unterschied zum Hustengerät kann das Beatmungsgerät zusätzliche Beatmungsfunktionen zum druck- und/oder fluss- und/oder volumengesteuerten Beatmen eines Patienten umfassen.

Im Folgenden werden verschiedene Ausführungsformen der Erfindung beschrieben. Diese Ausführungsformen sind nicht als Beschränkung des Umfangs der Erfindung zu verstehen.

Gemäß einer Ausführungsform kann die Drosseleinrichtung mindestens so lange in der Drosselstellung gehalten werden, bis die Koppeleinrichtung in die Exsufflationsstellung geschaltet wird. Dies ermöglicht die Bereitstellung eines entsprechend erhöhten Volumenstroms zu Beginn der Exsufflation, was die Sekretabfuhr verbessern kann.

Gemäß einer Ausführungsform kann die Drosseleinrichtung im Insufflationsverfahren unter Berücksichtigung eines zeitlichen Verlaufs des Insufflationsdrucks und/oder eines zeitlichen Verlaufs der Motordrehzahl in die Drosselstellung geschaltet und/oder in der Drosselstellung gehalten werden. Auf diese Weise kann ein geeigneter Schaltzeitpunkt zum Umschalten der Drosseleinrichtung zwischen der Ausgangsstellung und der Drosselstellung abhängig von aktuellen Betriebsbedingungen des Hustengeräts bestimmt werden. Alternativ kann der Schaltzeitpunkt fest vorgegeben sein, beispielsweise abhängig von einer (vorgegebenen) Dauer des Insufflationsverfahrens.

Gemäß einer Ausführungsform kann die Drosseleinrichtung in die Drosselstellung geschaltet werden, wenn mindestens eine der folgenden Bedingungen erfüllt ist:
- der Insufflationsdruck beträgt mindestens für eine vorgegebene Dauer mindestens 80 %, vorzugsweise mindestens 90 % des Insufflationssolldrucks;
- der Insufflationssolldruck ist mindestens für eine vorgegebene Dauer konstant;
- die Motordrehzahl liegt mindestens für eine vorgegebene Dauer unterhalb eines Drehzahlschwellenwerts, der unter Berücksichtigung einer maximal möglichen Motordrehzahl so gewählt ist, dass ein beim Schalten der Drosseleinrichtung in die Drosselstellung auftretender Abfall des Insufflationsdrucks durch Erhöhen der Motordrehzahl über den Drehzahlschwellenwert hinaus gerade noch kompensiert werden kann.

Gemäß einer Ausführungsform kann das Insufflationsverfahren ferner umfassen: Verhindern, dass die Drosseleinrichtung in die Drosselstellung geschaltet wird, wenn mindestens eine der folgenden Bedingungen erfüllt ist:
- der Insufflationssolldruck ist betragsmäßig größer als ein Exsufflationssolldruck, der bei einer Exsufflation am Patientenanschluss anliegen soll;
- der Insufflationssolldruck liegt über einem Druckschwellenwert, der unter Berücksichtigung eines maximal möglichen Insufflationsdrucks so gewählt ist, dass ein beim Schalten der Drosseleinrichtung in die Drosselstellung auftretender Abfall des Insufflationsdrucks in Höhe des Druckschwellenwerts durch Erhöhen der Motordrehzahl gerade noch kompensiert werden kann;
- eine Funktion zum Bewirken einer gezielten Oszillation des Insufflationsdrucks ist aktiviert.

Unter "Exsufflationsdruck" kann ein Unterdruck oder negativer Druck relativ zum jeweiligen Atmosphärendruck verstanden werden.

Gemäß einer Ausführungsform kann die Drosseleinrichtung in mindestens eine zusätzliche Drosselstellung schaltbar sein und ausgebildet sein, um den Verbindungskanal beim Schalten in die zusätzliche Drosselstellung anders als beim Schalten in die Drosselstellung zu verengen. In diesem Fall kann die Drosseleinrichtung beim Umschalten zwischen der Ausgangsstellung und der Drosselstellung die zusätzliche Drosselstellung passieren. Es ist möglich, dass der Verbindungskanal - wenn die Drosseleinrichtung in die zusätzliche Drosselstellung geschaltet ist - stärker verengt ist, als wenn die Drosseleinrichtung in die Drosselstellung geschaltet ist (oder umgekehrt). Auf diese Weise kann der Verbindungskanal kontinuierlich und/oder stufenweise, beispielsweise degressiv und/oder progressiv, verengt werden. Dies ermöglicht eine bessere Steuerung der Verengung des Verbindungskanals gegenüber einer Ausführungsform mit nur einer Drosselstellung. Somit können unerwünschte Schwankungen des Insufflationsdrucks beim Verengen des Verbindungskanals vermieden werden.

Gemäß einer Ausführungsform kann die Drosseleinrichtung beim Schalten in die Drosselstellung zunächst mit einer ersten Schaltgeschwindigkeit (oder gemäß einem vorgegebenen ersten Schaltgeschwindigkeitsverlauf) von der Ausgangsstellung in die zusätzliche Drosselstellung und anschließend mit einer von der ersten Schaltgeschwindigkeit abweichenden zweiten Schaltgeschwindigkeit (oder gemäß einem vom ersten Schaltgeschwindigkeitsverlauf abweichenden vorgegebenen zweiten Schaltgeschwindigkeitsverlauf) von der zusätzlichen Drosselstellung in die Drosselstellung bewegt werden. Dabei kann die erste Schaltgeschwindigkeit (im Schnitt) deutlich größer als die zweite Schaltgeschwindigkeit sein (oder umgekehrt). Anders ausgedrückt ist es möglich, dass die Drosseleinrichtung beim Schalten in die Drosselstellung zunächst sprunghaft in die zusätzliche Drosselstellung und anschließend deutlich langsamer in die Drosselstellung bewegt wird. Eine solche Ausführungsform mit unterschiedlichen Schaltgeschwindigkeiten erwies sich in Versuchen als besonders günstig.

Gemäß einer Ausführungsform kann die Steuereinrichtung ausgebildet sein, um ferner folgendes Exsufflationsverfahren, beispielsweise (unmittelbar) nach dem Insufflationsverfahren und/oder (automatisch) als Reaktion auf ein Beenden des Insufflationsverfahrens, auszuführen: Schalten der Koppeleinrichtung in die Exsufflationsstellung; Schalten der Drosseleinrichtung in die Ausgangsstellung; (wenn die Koppeleinrichtung in die Exsufflationsstellung geschaltet wurde:) Empfangen des Drucksignals, wobei das Drucksignal einen am Patientenanschluss anliegenden Exsufflationsdruck als den erfassten Druck anzeigt; Bestimmen einer Exsufflationsdruckabweichung zwischen dem Exsufflationsdruck und einem Exsufflationssolldruck; Steuern des Antriebsmotors, um die Exsufflationsdruckabweichung zu verringern. Das Schalten der Koppeleinrichtung kann gleichzeitig mit oder (beispielsweise geringfügig) zeitlich versetzt zu (beispielsweise vor und/oder nach) dem Schalten der Drosseleinrichtung erfolgen. Das Umschalten zwischen dem Insufflationsverfahren und dem Exsufflationsverfahren kann automatisch und/oder manuell erfolgen. Zweckmäßigerweise kann die Steuereinheit ausgebildet sein, um im Betrieb des Hustengeräts (automatisch) mehrmals abwechselnd zwischen dem Insufflationsverfahren und dem Exsufflationsverfahren umzuschalten.

Gemäß einer Ausführungsform kann die Koppeleinrichtung ferner in mindestens eine Zwischenstellung schaltbar sein und ausgebildet sein, um den Druckanschluss in der Zwischenstellung sowohl mit der Ansaugöffnung als auch mit der Ausblasöffnung fluidisch zu koppeln. In diesem Fall kann das Exsufflationsverfahren ferner umfassen: Schalten der Koppeleinrichtung von der Exsufflationsstellung in die Zwischenstellung, um unerwünschte Schwankungen des Exsufflationsdrucks zu dämpfen. Durch die erhöhte Motordrehzahl beim Schalten in die Exsufflationsstellung können unter Umständen unerwünschte Druckschwankungen auftreten. Um diesen Effekt ohne nennenswerten Einfluss auf die Druckregelung abzumildern, kann die Koppeleinrichtung nach dem Erreichen der Exsufflationsstellung kurz in die Zwischenstellung geschaltet werden. Die Koppeleinrichtung kann auch mehrmals zwischen der Exsufflationsstellung und der Zwischenstellung und/oder zwischen verschiedenen Zwischenstellungen umgeschaltet werden, um die unerwünschten Druckschwankungen zu dämpfen. Die Koppeleinrichtung kann beispielsweise ausgebildet sein, um den Verbindungskanal in der Zwischenstellung zusätzlich zu belüften, sodass ein zusätzlicher Druckausgleich mit einer Umgebung des Verbindungskanals erfolgt.

Gemäß einer Ausführungsform kann die Koppeleinrichtung unter Berücksichtigung eines zeitlichen Verlaufs des Exsufflationsdrucks und/oder eines zeitlichen Verlaufs der Motordrehzahl in die Zwischenstellung geschaltet und/oder in der Zwischenstellung gehalten werden. Auf diese Weise kann ein geeigneter Schaltzeitpunkt zum Umschalten der Koppeleinrichtung zwischen der Exsufflationsstellung und der Zwischenstellung abhängig von aktuellen Betriebsbedingungen des Hustengeräts bestimmt werden. Alternativ kann der Schaltzeitpunkt fest vorgegeben sein, beispielsweise abhängig von einer (vorgegebenen) Dauer des Exsufflationsverfahrens.

Gemäß einer Ausführungsform kann eine Amplitude des Drucksignals und/oder eines die Motordrehzahl anzeigenden Drehzahlsignals in mehreren aufeinanderfolgenden Zeitschritten mit einem vorgegebenen Toleranzbereich, zwischen dessen Grenzen die Amplitude schwanken darf, verglichen werden, wobei die Koppeleinrichtung abhängig von einer Abweichung der Amplitude von mindestens einer der Grenzen zwischen der Exsufflationsstellung und der Zwischenstellung umgeschaltet werden kann. Der Toleranzbereich kann beispielsweise eine Schwankungsbreite in einer Größenordnung von plus/minus 20 %, plus/minus 10 %, plus/minus 5 % oder plus/minus 1 % um einen gewünschten Durchschnittswert der Amplitude definieren. Wird beispielsweise erkannt, dass die Amplitude mindestens für eine vorgegebene Dauer im Toleranzbereich liegt, so kann die Koppeleinrichtung in die Exsufflationsstellung zurückgeschaltet und bis zum Ende des Exsufflationsverfahrens in der Exsufflationsstellung gehalten werden.

Alternativ kann die Koppeleinrichtung in einem fest vorgegebenen Zeitfenster in der Zwischenstellung gehalten werden.

Gemäß einer Ausführungsform kann die Drosseleinrichtung ferner zwischen einer ersten Oszillationsstellung und einer zweiten Oszillationsstellung umschaltbar sein und ausgebildet sein, um den Verbindungskanal beim Schalten von der ersten Oszillationsstellung in die zweite Oszillationsstellung zu verengen. In diesem Fall kann im In- und/oder Exsufflationsverfahren ferner folgender Schritt ausgeführt werden: Abwechselndes Umschalten der Drosseleinrichtung zwischen der ersten Oszillationsstellung und der zweiten Oszillationsstellung, um eine gezielte Oszillation des In- bzw. Exsufflationsdrucks zu bewirken. Dies kann die Sekretabfuhr weiter verbessern. Das abwechselnde Umschalten kann nach dem (erstmaligen) Schalten der Drosseleinrichtung in die Ausgangsstellung und/oder unter Berücksichtigung eines zeitlichen Verlaufs des In- bzw. Exsufflationsdrucks und/oder eines zeitlichen Verlaufs der Motordrehzahl erfolgen. Beispielsweise kann das abwechselnde Umschalten erst dann erfolgen, wenn erkannt wird, dass der (gemessene) In- bzw. Exsufflationsdruck oder der In- bzw. Exsufflationssolldruck mindestens für eine vorgegebene Dauer konstant ist. Unter "Oszillationsstellung" kann vor- und nachstehend eine fest vorgegebene oder im Betrieb des Hustengeräts variierbare Schaltstellung der Drosseleinrichtung verstanden werden. Die Schaltstellung kann beispielsweise abhängig vom zeitlichen Verlauf des Drucksignals und/oder eines die Motordrehzahl anzeigenden Drehzahlsignals und/oder abhängig von einem vorgegebenen Oszillationsverlauf variierbar sein.

Gemäß einer Ausführungsform kann die erste Oszillationsstellung die Ausgangsstellung sein. Alternativ kann die erste Oszillationsstellung von der Ausgangsstellung abweichen und beispielweise eine zwischen der Ausgangsstellung und der Drosselstellung befindliche Stellung sein.

Gemäß einer Ausführungsform kann die zweite Oszillationsstellung die Drosselstellung sein. Alternativ kann die zweite Oszillationsstellung von der Drosselstellung abweichen.

Anders ausgedrückt kann die Drosseleinrichtung ausgebildet sein, um den Verbindungskanal beim Schalten in die zweite Oszillationsstellung anders als beim Schalten in die Drosselstellung (und/oder anders als beim Schalten in die vorgenannte zusätzliche Drosselstellung) zu verengen. So kann der Verbindungskanal - wenn die Drosseleinrichtung in die zweite Oszillationsstellung geschaltet ist - stärker verengt sein, als wenn die Drosseleinrichtung in die Drosselstellung geschaltet ist (oder umgekehrt). Es ist möglich, dass die Drosseleinrichtung beim Umschalten zwischen der ersten Oszillationsstellung und der zweiten Oszillationsstellung die Drosselstellung (und gegebenenfalls die vorgenannte zusätzliche Drosselstellung) passiert. Dies ermöglicht es beispielsweise, den Verbindungskanal kontinuierlich und/oder stufenweise, beispielsweise degressiv und/oder progressiv, zu verengen bzw. zu erweitern, um die gezielte Oszillation zu bewirken.

Soll eine gezielte Oszillation des Insufflationsdrucks im Insufflationsverfahren bewirkt werden, so ist es zweckmäßig, wenn das Schalten der Drosseleinrichtung in die Drosselstellung zum Zweck der zusätzlichen Erhöhung der Motordrehzahl unterbunden wird.

Gemäß einer Ausführungsform kann das Hustengerät ferner einen Flusssensor umfassen, der ausgebildet ist, um einen Volumenstrom durch den Verbindungskanal zu erfassen und ein den erfassten Volumenstrom anzeigendes Flusssignal zu erzeugen. In diesem Fall kann die Steuereinrichtung ausgebildet sein, um ferner das Flusssignal zum Schalten der Koppeleinrichtung und/oder der Drosseleinrichtung und/oder zum Steuern des Antriebsmotors auszuwerten. Der Flusssensor kann beispielsweise ausgebildet sein, um einen Volumenstrom durch einen zwischen der Drosseleinrichtung und dem Patientenanschluss befindlichen Abschnitt des Verbindungskanals zu erfassen. Dies ermöglicht eine genauere Steuerung des Betriebs des Hustengeräts im Vergleich zu einer Ausführungsform ohne Berücksichtigung des Volumenstroms.

Gemäß einer Ausführungsform kann das Exsufflationsverfahren ferner umfassen: Empfangen des Flusssignals, wobei das Flusssignal einen exsufflierten Volumenstrom als den erfassten Volumenstrom anzeigt, und Auswerten des Flusssignals, um ein lokales Maximum im zeitlichen Verlauf des erfassten Volumenstroms zu erkennen. In diesem Fall kann das abwechselnde Umschalten zum Bewirken der gezielten Oszillation als Reaktion auf das Erkennen des lokalen Maximums, beispielsweise des Peak Cough Flow, erfolgen.

Gemäß einer Ausführungsform kann die Drosseleinrichtung ein um eine Drehachse zwischen der Ausgangsstellung und der Drosselstellung drehbar gelagertes Stellglied und einen elektrischen oder elektrisch steuerbaren Aktor zum Drehen des Stellglieds umfassen. Das Stellglied kann einen Wandabschnitt zum Verringern eines Strömungsquerschnitts des Verbindungskanals umfassen und so ausgebildet sein, dass der Wandabschnitt in der Drosselstellung weiter in den Verbindungskanal hineinragt als in der Ausgangsstellung.

Gemäß einer Ausführungsform kann das Hustengerät ferner einen in den Verbindungskanal mündenden Belüftungskanal zum Ermöglichen eines Druckausgleichs zwischen dem Inneren des Verbindungskanals und einer äußeren Umgebung des Verbindungskanals umfassen. In diesem Fall kann die Drosseleinrichtung ausgebildet sein, um den Belüftungskanal in der Ausgangsstellung zu verschließen und in der Drosselstellung freizugeben. Dies kann so verstanden werden, dass der Belüftungskanal - wenn die Drosseleinrichtung in die Ausgangsstellung geschaltet ist - stärker verengt ist, als wenn die Drosseleinrichtung in die Drosselstellung geschaltet ist. Beispielsweise kann der Belüftungskanal in der Ausgangsstellung entweder vollständig verengt, d. h. gasdicht verschlossen sein oder teilweise (beispielsweise um mindestens 5 % und/oder höchstens 95 % relativ zur Drosselstellung) verengt sein, sodass weiterhin Gas durch den Belüftungskanal strömen kann. Dies ermöglicht es, beim Schalten der Drosseleinrichtung in die Drosselstellung eine geringfügige interne Leckage zu erzeugen, was sich günstig auf den Druckverlauf auswirken kann.

Zusätzlich kann die Drosseleinrichtung ausgebildet sein, um den Belüftungskanal in der ersten Oszillationsstellung zu verschließen und/oder in der zweiten Oszillationsstellung freizugeben.

### Kurze Beschreibung der Zeichnungen

Im Folgenden werden Ausführungsformen der Erfindung mit Bezug auf die beigefügten Zeichnungen beschrieben. Weder die Beschreibung noch die Zeichnungen sind als Beschränkung des Umfangs der Erfindung zu verstehen.
Fig. 1 zeigt ein Hustengerät gemäß einer Ausführungsform der Erfindung mit einer in einer Insufflationsstellung befindlichen Koppeleinrichtung und einer in einer Ausgangsstellung befindlichen Drosseleinrichtung.
Fig. 2 zeigt das Hustengerät aus Fig. 1, wobei sich die Drosseleinrichtung in einer ersten Drosselstellung befindet.
Fig. 3 zeigt das Hustengerät aus Fig. 1, wobei sich die Drosseleinrichtung in einer zweiten Drosselstellung befindet.
Fig. 4 zeigt das Hustengerät aus Fig. 1, wobei sich die Koppeleinrichtung in einer Exsufflationsstellung befindet.
Fig. 5 zeigt das Hustengerät aus Fig. 4, wobei sich die Koppeleinrichtung in einer Zwischenstellung befindet.
Fig. 6 zeigt das Hustengerät aus Fig. 4, wobei sich die Drosseleinrichtung in einer Oszillationsstellung befindet.
Fig. 7 zeigt ein Diagramm zur Veranschaulichung eines zeitlichen Verlaufs eines Drucks und eines Volumenstroms beim Betrieb eines Hustengeräts gemäß einer Ausführungsform der Erfindung.

Die Figuren sind rein schematisch und nicht maßstabsgetreu. Werden in verschiedenen Zeichnungen gleiche Bezugszeichen verwendet, so bezeichnen diese Bezugszeichen gleiche oder gleichwirkende Merkmale.

### Ausführungsformen der Erfindung

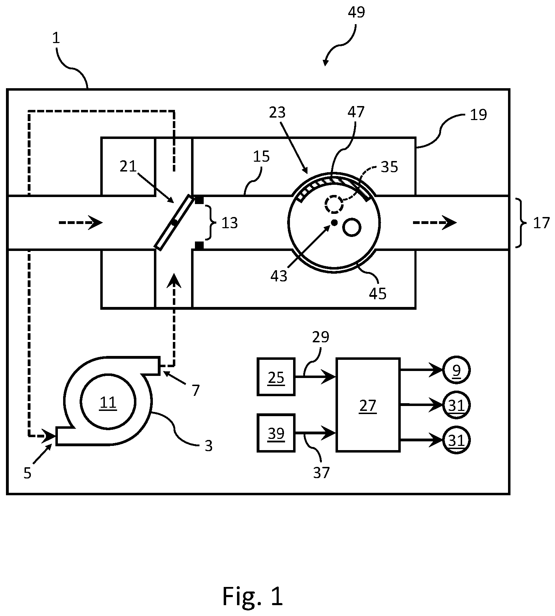

Fig. 1 zeigt ein Hustengerät 1 zum Unterstützen eines Patienten beim Husten. Das Hustengerät 1 umfasst ein Gebläse 3 mit einer Ansaugöffnung 5, einer Ausblasöffnung 7, einem Antriebsmotor 9 und einem mit dem Antriebsmotor 9 gekoppelten Rotor 11 zum Fördern eines Gases von der Ansaugöffnung 5 zur Ausblasöffnung 7 abhängig von einer Motordrehzahl des Antriebsmotors 7. Eine Strömungsrichtung des Gases ist mit gestrichelten Pfeilen markiert.

Des Weiteren umfasst das Hustengerät 1 einen Druckanschluss 13 und einen über einen Verbindungskanal 15 mit dem Druckanschluss 13 fluidisch verbundenen Patientenanschluss 17 zum Anschließen einer Patientenschnittstelle, beispielsweise einer Maske, eines Mundstücks, einer Endotrachealkanüle oder einer Tracheostomiekanüle. Die beiden Anschlüsse 13, 15 können beispielsweise als Anschlüsse einer Pneumatikeinheit 19 ausgebildet sein. Die Pneumatikeinheit 19 kann beispielsweise als ein Ventilgehäuse oder Ventilblock ausgebildet sein.

Darüber hinaus umfasst das Hustengerät 1 eine Koppeleinrichtung 21, eine Drosseleinrichtung 23, einen Drucksensor 25 und eine Steuereinrichtung 27 zum Steuern eines Betriebs des Hustengeräts 1.

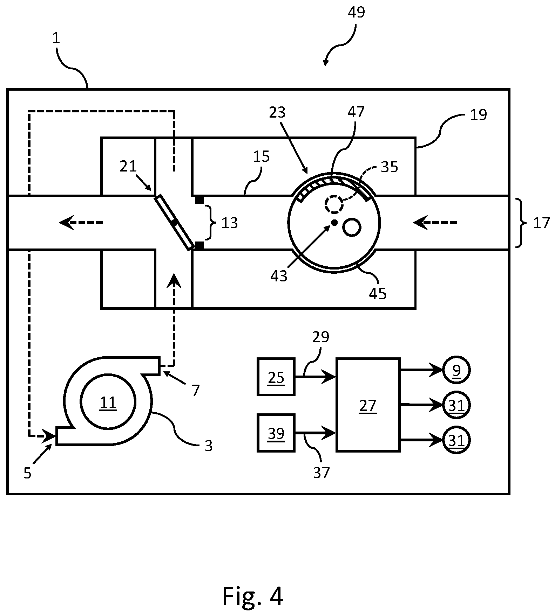

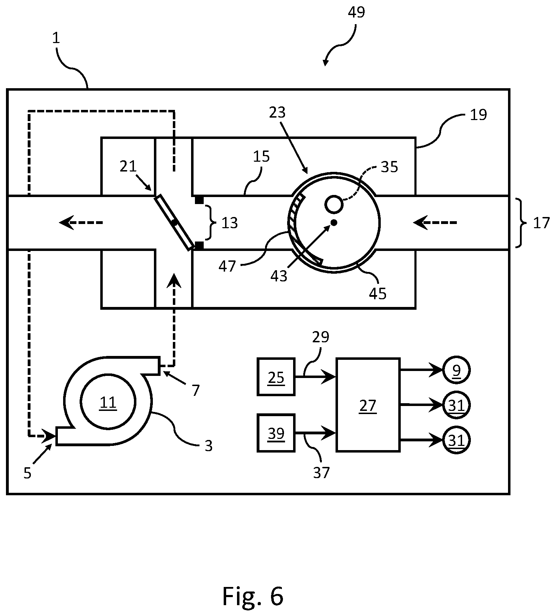

Die Koppeleinrichtung 21, hier in Form eines Klappenventils, ist zwischen einer Insufflationsstellung und einer Exsufflationsstellung umschaltbar und ausgebildet, um den Druckanschluss 13 in der Insufflationsstellung (siehe Fig. 1 bis Fig. 3) mit der Ausblasöffnung 7 und in der Exsufflationsstellung (siehe Fig. 4 und Fig. 6) mit der Ansaugöffnung 5 fluidisch zu koppeln.

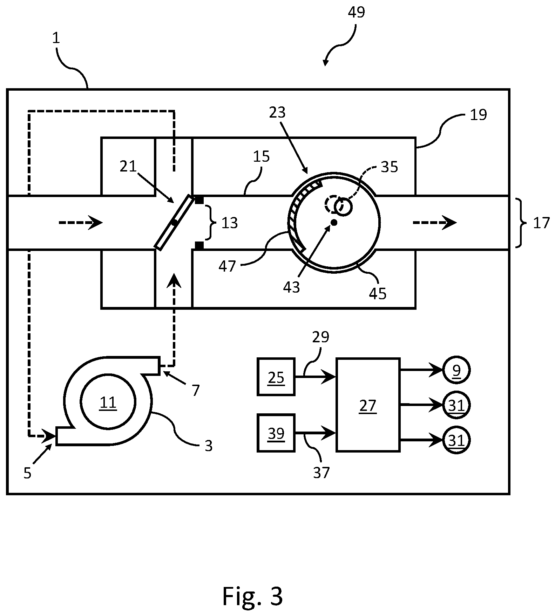

Die Drosseleinrichtung 23 ist zwischen einer Ausgangsstellung (siehe Fig. 1 und Fig. 4) und einer Drosselstellung (siehe Fig. 3) umschaltbar und ausgebildet, um den Verbindungskanal 15 beim Schalten von der Ausgangsstellung in die Drosselstellung gezielt zu verengen.

Der Drucksensor 25 ist ausgebildet, um einen am Patientenanschluss 17 anliegenden Druck zu erfassen und ein den erfassten Druck anzeigendes Drucksignal 29 zu erzeugen.

Die Steuereinrichtung 27 ist ausgebildet, um folgendes Insufflationsverfahren auszuführen: Schalten der Koppeleinrichtung 21 in die Insufflationsstellung; Empfangen des Drucksignals 29, wobei das Drucksignal 29 einen am Patientenanschluss 17 anliegenden Insufflationsdruck als den erfassten Druck anzeigt; Bestimmen einer Insufflationsdruckabweichung zwischen dem Insufflationsdruck und einem Insufflationssolldruck, der beispielsweise manuell eingestellt wurde; Steuern des Antriebsmotors 9, um die Insufflationsdruckabweichung zu verringern; beim Steuern des Antriebsmotors 9: Schalten der Drosseleinrichtung 23 in die Drosselstellung und Halten der Drosseleinrichtung 23 in der Drosselstellung, um eine zusätzliche Erhöhung der Motordrehzahl zu bewirken.

Beispielsweise kann die Drosseleinrichtung 23 so lange in der Drosselstellung gehalten werden, bis die Koppeleinrichtung 21 in die Exsufflationsstellung geschaltet wird, um eine Exsufflation einzuleiten. Denkbar ist auch, dass die Drosseleinrichtung 23 kurzzeitig bis über den Zeitpunkt des Umschaltens in die Exsufflationsstellung hinaus in der Drosselstellung gehalten wird. Alternativ ist es möglich, dass die Drosseleinrichtung 23 bereits kurz vor dem Zeitpunkt des Umschaltens in die Exsufflationsstellung zurück in die Ausgangsstellung geschaltet wird.

Dies ermöglicht eine deutliche Steigerung des PCF, ohne dass ein leistungsstärkeres Gebläse oder eine schnellere Umschaltung von der Insufflations- in die Exsufflationsstellung erforderlich ist. Die Drosselung, speziell zum Ende der Insufflation hin, hat die Wirkung, dass ein dem Gebläse bei der Insufflation entgegenwirkender Strömungswiderstand zusätzlich erhöht wird. Aufgrund der Druckregelung erhöht sich die Motordrehzahl entsprechend. Dies ermöglicht es, in der anschließenden Exsufflation einen entsprechend erhöhten Volumenstrom bereitzustellen.

Es ist möglich, dass die Koppeleinrichtung 21 und die Drosseleinrichtung 23 jeweils einen elektrischen oder elektrisch steuerbaren Aktor 31 zum Umschalten zwischen den jeweiligen Schaltstellungen umfassen. Ein solcher Aktor 31 kann beispielsweise ein Elektromotor, ein Elektromagnet, ein Piezoelement oder eine Kombination von mindestens zwei dieser Beispiele sein.

Zusätzlich kann die Steuereinrichtung 27 ausgebildet sein, um folgendes Exsufflationsverfahren im Anschluss an das Insufflationsverfahren auszuführen: Schalten der Koppeleinrichtung 21 in die Exsufflationsstellung; Schalten der Drosseleinrichtung 23 zurück in die Ausgangsstellung, beispielsweise zeitgleich mit dem Schalten der Koppeleinrichtung 21 in die Exsufflationsstellung; Empfangen des Drucksignals 29, wobei das Drucksignal 29 einen am Patientenanschluss 17 anliegenden Exsufflationsdruck als den erfassten Druck anzeigt; Bestimmen einer Exsufflationsdruckabweichung zwischen dem Exsufflationsdruck und einem Exsufflationssolldruck, der beispielsweise ebenfalls manuell eingestellt wurde; Steuern des Antriebsmotors 9, um die Exsufflationsdruckabweichung zu verringern.

Das Umschalten zwischen dem Insufflationsverfahren und dem Exsufflationsverfahren kann manuell und/oder automatisch erfolgen.

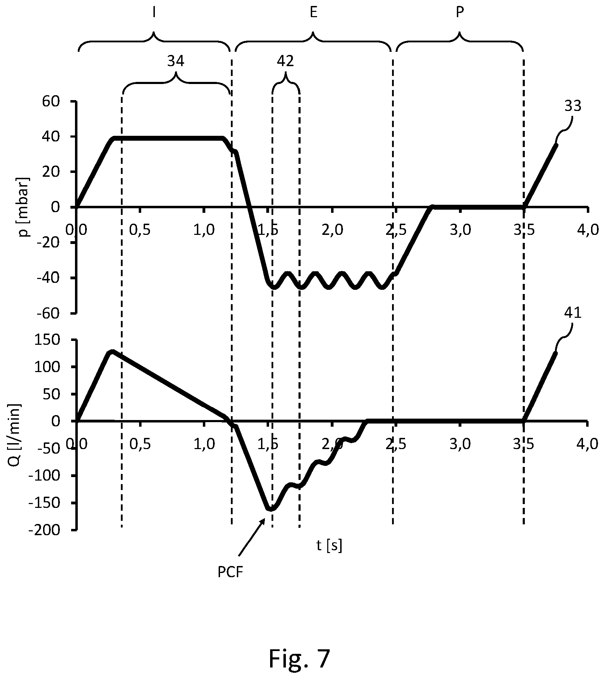

Beispielsweise kann die Steuereinrichtung 27 ausgebildet sein, um das Hustengerät 1 zwischen zwei Hustenzyklen in eine Pausenstellung (siehe den Abschnitt "P" in Fig. 7) zu schalten, wobei das Hustengerät 1 so ausgebildet ist, dass in der Pausenstellung ein jeweiliger Atmosphärendruck am Patientenanschluss 17 anliegt.

Es ist zweckmäßig, wenn die Drosseleinrichtung 23 im Insufflationsverfahren unter Berücksichtigung eines zeitlichen Verlaufs 33 (siehe Fig. 7) des Insufflationsdrucks und/oder eines zeitlichen Verlaufs der Motordrehzahl in die Drosselstellung geschaltet und/oder in der Drosselstellung gehalten wird. Dies ermöglicht es, die Drosseleinrichtung 23 abhängig von aktuellen Betriebsbedingungen des Hustengeräts 1 zu steuern. Die Drosseleinrichtung 23 kann aber auch zu einem fest vorgegebenen Schaltzeitpunkt umgeschaltet werden. In Fig. 7 ist beispielhaft ein mögliches Drosselungszeitfenster 34, in dem die Drosseleinrichtung 23 in der Drosselstellung gehalten wird, markiert.

Die Steuereinrichtung 27 kann ausgebildet sein, um die Drosseleinrichtung 23 abhängig davon, ob bestimmte Schaltbedingungen erfüllt sind, in die Drosselstellung zu schalten.

Die Schaltbedingungen können beispielsweise folgende notwendige Bedingungen umfassen:
- der Insufflationssolldruck ist betragsmäßig höchstens so groß wie der Exsufflationssolldruck (andernfalls wird der Antriebsmotor 9 im Insufflationsverfahren ohnehin mit einer höheren Motordrehzahl als im Exsufflationsverfahren betrieben);
- der Insufflationssolldruck ist höchstens so groß wie ein vorgegebener Druckschwellenwert, der unter Berücksichtigung eines maximal möglichen Insufflationsdrucks so gewählt ist, dass ein beim Schalten der Drosseleinrichtung 23 in die Drosselstellung auftretender Abfall des Insufflationsdrucks in Höhe des Druckschwellenwerts durch Erhöhen der Motordrehzahl gerade noch kompensiert werden kann (überschreitet der Insufflationssolldruck den Druckschwellenwert, so ist unter Umständen keine ausreichende Drehzahlreserve mehr vorhanden);
- eine etwaige Funktion zum Bewirken einer gezielten Oszillation des Insufflationsdrucks ist deaktiviert.

Je nach Ausführungsform des Hustengeräts 1, insbesondere des Gebläses 3, kann der Druckschwellenwert beispielsweise zwischen 40 hPa und 70 hPa, insbesondere zwischen 50 hPa und 60 hPa, liegen.

Trifft mindestens eine der notwendigen Bedingungen nicht zu, so unterbindet die Steuereinrichtung 27 das Schalten der Drosseleinrichtung 23 in die Drosselstellung während der Insufflation.

Zudem können die Schaltbedingungen beispielsweise folgende hinreichende Bedingungen umfassen:
- der (gemessene) Insufflationsdruck beträgt mindestens für eine vorgegebene Dauer mindestens 80 %, vorzugsweise mindestens 90 % des Insufflationssolldrucks;
- der Insufflationssolldruck ist mindestens für eine vorgegebene Dauer konstant (d. h., der Insufflationssolldruck hat eine vorgegebene Plateauphase erreicht);
- die Motordrehzahl liegt mindestens für eine vorgegebene Dauer unterhalb eines vorgegebenen Drehzahlschwellenwerts, der unter Berücksichtigung einer maximal möglichen Motordrehzahl so gewählt ist, dass ein beim Schalten der Drosseleinrichtung 23 in die Drosselstellung auftretender Abfall des Insufflationsdrucks durch Erhöhen der Motordrehzahl über den Drehzahlschwellenwert hinaus gerade noch kompensiert werden kann.

Trifft jede der hinreichenden Bedingungen zu, so schaltet die Steuereinrichtung 27 die Drosseleinrichtung 23 während der Insufflation in die Drosselstellung.

Je nach Ausführungsform des Hustengeräts 1, insbesondere des Gebläses 3, kann der Drehzahlschwellenwert beispielsweise zwischen 20.000 und 60.000, insbesondere zwischen 35.000 und 45.000, vorzugsweise bei 42.000 Umdrehungen pro Minute liegen.

Die vorgegebene Dauer kann beispielsweise zwischen 0,1 s und 1 s, vorzugsweise zwischen 0,4 s und 0,7 s, insbesondere 0,5 s, betragen.

Das Prüfen, ob die vorgenannten Bedingungen erfüllt sind, kann in mehreren aufeinanderfolgenden Zeitschritten erfolgen, beispielsweise zwischen 50-mal und 200-mal pro Sekunde, insbesondere zwischen 90-mal und 110-mal pro Sekunde.

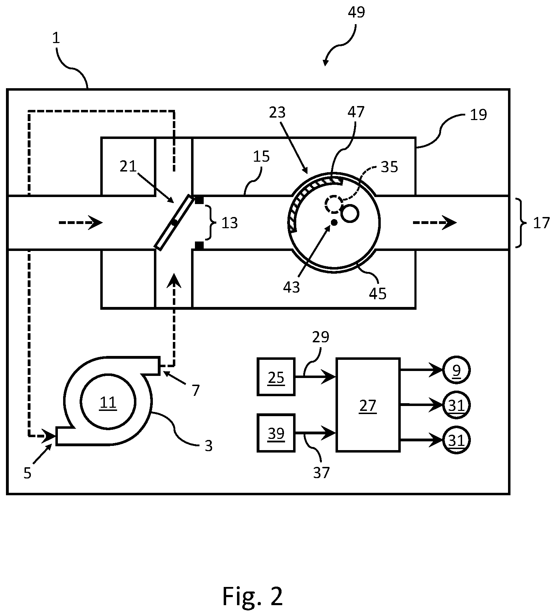

Wie in Fig. 2 gezeigt, kann die Drosseleinrichtung 23 in mindestens eine zwischen der Ausgangsstellung und der Drosselstellung befindliche zusätzliche Drosselstellung schaltbar sein und ausgebildet sein, um den Verbindungskanal 15 beim Schalten in die zusätzliche Drosselstellung anders, beispielsweise stärker, als beim Schalten in die Drosselstellung zu verengen. Dabei startet das Insufflationsverfahren mit der Ausgangsstellung. Sind die vorgenannten Bedingungen erfüllt, so wird beispielsweise die Drosseleinrichtung 23 zunächst sprunghaft in die zusätzliche Drosselstellung geschaltet, in der ein dem Gebläse 3 entgegenwirkender Strömungswiderstand im Vergleich zur Ausgangsstellung deutlich erhöht ist. Anschließend wird die Drosseleinrichtung 23 mit verringerter Stellgeschwindigkeit von der zusätzlichen Drosselstellung bis in die Drosselstellung bewegt. Beispielsweise kann die Drosseleinrichtung 23 ausgebildet sein, um einen in den Verbindungskanal 15 mündenden Belüftungskanal 35 in der Ausgangsstellung (und gegebenenfalls in der zusätzlichen Drosselstellung) luftdicht zu verschließen und in der Drosselstellung teilweise freizugeben, sodass in der Drosselstellung ein zusätzlicher Druckausgleich mit einer Umgebung des Verbindungskanals 15 erfolgt. Dies kann sich günstig auf den Druckverlauf auswirken.

Zusätzlich kann die Drosseleinrichtung 23 zwischen einer ersten Oszillationsstellung und einer zweiten Oszillationsstellung umschaltbar sein und ausgebildet sein, um den Verbindungskanal 15 beim Schalten von der ersten Oszillationsstellung in die zweite Oszillationsstellung zu verengen. In diesem Fall ist es möglich, dass die Drosseleinrichtung 23 im In- und/oder Exsufflationsverfahren mehrmals abwechselnd zwischen der ersten Oszillationsstellung und der zweiten Oszillationsstellung umgeschaltet wird, um eine gezielte Oszillation des In- bzw. Exsufflationsdrucks zu bewirken.

In diesem Beispiel stimmt die erste Oszillationsstellung mit der Ausgangsstellung überein, während es sich bei der zweiten Oszillationsstellung (siehe Fig. 6) um eine von der Drosselstellung bzw. von den verschiedenen Drosselstellungen abweichende Stellung handelt, in der die Drosseleinrichtung 23 den Belüftungskanal 35 - im Gegensatz zur Drosselstellung bzw. zu den Drosselstellungen - vollständig freigibt.

Es ist möglich, dass die Steuereinrichtung 27 zusätzlich zum Drucksignal 29 ein Flusssignal 37 von einem Flusssensor 39 zum Erfassen eines Volumenstroms durch einen zwischen der Drosseleinrichtung 23 und dem Patientenanschluss 17 befindlichen Abschnitt des Verbindungkanals 15 empfängt. In diesem Fall kann das abwechselnde Umschalten zwischen der ersten Oszillationsstellung und der zweiten Oszillationsstellung im Exsufflationsverfahren beispielsweise erst dann erfolgen, wenn anhand des Flusssignals 37 erkannt wird, dass ein exsufflierter Volumenstrom ein lokales Maximum, auch PCF genannt, erreicht hat. Fig. 7 zeigt beispielhaft einen zeitlichen Verlauf 41 des Volumenstroms im Betrieb des Hustengeräts 1.

Die Steuereinrichtung 27 kann auch ausgebildet sein, um das Flusssignal 37 zum Steuern des Antriebsmotors 9 und/oder der Koppeleinrichtung 21 auszuwerten.

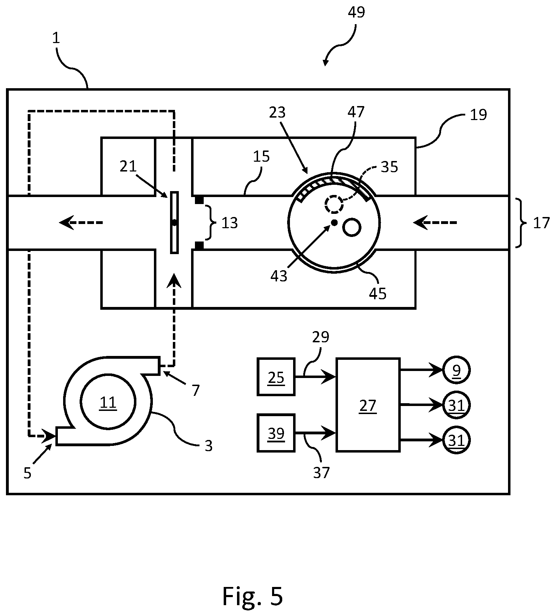

Um übermäßige Druckspitzen zu Beginn des Exsufflationsverfahrens zu dämpfen, kann die Koppeleinrichtung 21 zusätzlich in mindestens eine Zwischenstellung (siehe Fig. 5) schaltbar sein und ausgebildet sein, um den Druckanschluss 13 in der Zwischenstellung sowohl mit der Ansaugöffnung 5 als auch mit der Ausblasöffnung 7 fluidisch zu koppeln. In Fig. 7 ist beispielhaft ein mögliches Dämpfungszeitfenster 42 markiert, in dem die Koppeleinrichtung 21 in der Zwischenstellung gehalten oder abwechselnd zwischen der Exsufflationsstellung und der Zwischenstellung (und/oder zwischen verschiedenen Zwischenstellungen) umgeschaltet wird.

Beispielsweise kann die Steuereinrichtung 27 ausgebildet sein, um die Koppeleinrichtung 21 unter Berücksichtigung eines zeitlichen Verlaufs 33 des Exsufflationsdrucks und/oder eines zeitlichen Verlaufs der Motordrehzahl zwischen der Exsufflationsstellung und der Zwischenstellung (und/oder zwischen den verschiedenen Zwischenstellungen) umzuschalten. Dazu kann beispielsweise eine Amplitude des Drucksignals 29 und/oder eines die Motordrehzahl anzeigenden Drehzahlsignals in mehreren aufeinanderfolgenden Zeitschritten mit einem vorgegebenen Toleranzbereich, zwischen dessen Grenzen die Amplitude schwanken darf, verglichen werden. Dementsprechend kann die Koppeleinrichtung 21 abhängig von einer Abweichung der Amplitude von mindestens einer der Grenzen zwischen der Exsufflationsstellung und der Zwischenstellung (und/oder zwischen den verschiedenen Zwischenstellungen) umgeschaltet werden.

Die Drosseleinrichtung 23 kann beispielsweise ein um eine Drehachse 43 zwischen der Ausgangsstellung und einer Endstellung mittels des jeweiligen Aktors 31 drehbares Stellglied 45 mit einem Wandabschnitt 47 zum Verringern des Strömungsquerschnitts des Verbindungskanals 15 umfassen. Die Endstellung ist in diesem Beispiel die zweite Oszillationsstellung, wobei das Stellglied 45 beim Drehen zwischen der Ausgangsstellung (die hier mit der ersten Oszillationsstellung übereinstimmt) und der Endstellung die Drosselstellung (oder die verschiedenen Drosselstellungen) passiert. Dabei kann die Drosseleinrichtung 23 so ausgebildet sein, dass der Wandabschnitt 47 in der Endstellung und/oder in jeder Drosselstellung weiter in den Verbindungskanal 15 hineinragt als in der Ausgangsstellung.

Das Hustengerät 1 kann als ein eigenständiges Gerät oder als eine Komponente eines Beatmungsgeräts 49 zum invasiven und/oder nicht invasiven Beatmen des Patienten ausgebildet sein.

Abschließend wird darauf hingewiesen, dass Begriffe wie "aufweisen", "umfassen", "einschließen", "mit" usw. keine anderen Elemente oder Schritte ausschließen und unbestimmte Artikel wie "ein" oder "eine" keine Vielzahl ausschließen.

Ferner wird darauf hingewiesen, dass Merkmale oder Schritte, die mit Verweis auf eine der vorstehenden Ausführungsformen beschrieben sind, auch in Kombination mit Merkmalen oder Schritten, die mit Verweis auf andere der vorstehenden Ausführungsformen beschrieben sind, verwendet werden können.

Bezugszeichen in den Ansprüchen sind nicht als Beschränkung des Umfangs des durch die Ansprüche definierten Gegenstands zu verstehen.

### Liste der Bezugszeichen

- 1: Hustengerät
- 3: Gebläse
- 5: Ansaugöffnung
- 7: Ausblasöffnung
- 9: Antriebsmotor
- 11: Rotor
- 13: Druckanschluss
- 15: Verbindungskanal
- 17: Patientenanschluss
- 19: Pneumatikeinheit
- 21: Koppeleinrichtung
- 23: Drosseleinrichtung
- 25: Drucksensor
- 27: Steuereinrichtung
- 29: Drucksignal
- 31: Aktor
- 33: zeitlicher Verlauf eines am Patientenanschluss anliegenden Drucks
- 34: Drosselungszeitfenster
- 35: Belüftungskanal
- 37: Flusssignal
- 39: Flusssensor
- 41: zeitlicher Verlauf eines Volumenstroms durch den Verbindungskanal
- 42: Dämpfungszeitfenster
- 43: Drehachse
- 45: Stellglied
- 47: Wandabschnitt
- 49: Beatmungsgerät
- p: Druck
- t: Zeit
- E: Exsufflation
- I: Insufflation
- P: Pause
- PCF: Peak Cough Flow
- Q: Volumenstrom

## Patentansprüche

1. Hustengerät (1) zum Unterstützen eines Patienten beim Husten, wobei das Hustengerät (1) umfasst:
ein Gebläse (3) mit einer Ansaugöffnung (5), einer Ausblasöffnung (7), einem Antriebsmotor (9) und einem mit dem Antriebsmotor (9) gekoppelten Rotor (11) zum Fördern eines Gases von der Ansaugöffnung (5) zur Ausblasöffnung (7) abhängig von einer Motordrehzahl des Antriebsmotors (9);
einen Druckanschluss (13);
einen Patientenanschluss (17) zum Anschließen einer Patientenschnittstelle;
einen Verbindungskanal (15), der den Druckanschluss (13) mit dem Patientenanschluss (17) fluidisch verbindet;
eine Koppeleinrichtung (21), die zwischen einer Insufflationsstellung und einer Exsufflationsstellung umschaltbar ist und ausgebildet ist, um den Druckanschluss (13) in der Insufflationsstellung mit der Ausblasöffnung (7) und in der Exsufflationsstellung mit der Ansaugöffnung (5) fluidisch zu koppeln;
eine Drosseleinrichtung (23), die zwischen einer Ausgangsstellung und einer Drosselstellung umschaltbar ist und ausgebildet ist, um den Verbindungskanal (15) beim Schalten von der Ausgangsstellung in die Drosselstellung zu verengen;
einen Drucksensor (25), der ausgebildet ist, um einen am Patientenanschluss (17) anliegenden Druck (p) zu erfassen und ein den erfassten Druck (p) anzeigendes Drucksignal (29) zu erzeugen;
eine Steuereinrichtung (27) zum Steuern eines Betriebs des Hustengeräts (1), wobei die Steuereinrichtung (27) ausgebildet ist, um folgendes Insufflationsverfahren auszuführen:
Schalten der Koppeleinrichtung (21) in die Insufflationsstellung;
Empfangen des Drucksignals (29), wobei das Drucksignal (29) einen am Patientenanschluss (17) anliegenden Insufflationsdruck (p) als den erfassten Druck (p) anzeigt;
Bestimmen einer Insufflationsdruckabweichung zwischen dem Insufflationsdruck (p) und einem Insufflationssolldruck;
Steuern des Antriebsmotors (9), um die Insufflationsdruckabweichung zu verringern;
**dadurch gekennzeichnet, dass** das Insufflationsverfahren ferner umfasst:
Schalten der Drosseleinrichtung (23) von der Ausgangsstellung in die Drosselstellung und Halten der Drosseleinrichtung (23) in der Drosselstellung beim Steuern des Antriebsmotors (9), um eine zusätzliche Erhöhung der Motordrehzahl zu bewirken, wobei die derart erhöhte Motordrehzahl mindestens bis zum Zeitpunkt eines Umschaltens der Koppeleinrichtung (21) in die Exsufflationsstellung beibehalten wird, um eine Bereitstellung eines entsprechend erhöhten Volumenstroms zur Steigerung des Hustenspitzenflusses in einer anschließenden Exsufflation zu ermöglichen.

2. Hustengerät (1) nach Anspruch 1,
wobei die Drosseleinrichtung (23) mindestens so lange in der Drosselstellung gehalten wird, bis die Koppeleinrichtung (21) in die Exsufflationsstellung geschaltet wird; und/oder
wobei die Drosseleinrichtung (23) unter Berücksichtigung eines zeitlichen Verlaufs (33) des Insufflationsdrucks (p) und/oder eines zeitlichen Verlaufs der Motordrehzahl in die Drosselstellung geschaltet und/oder in der Drosselstellung gehalten wird.

3. Hustengerät (1) nach einem der vorhergehenden Ansprüche,
wobei die Drosseleinrichtung (23) in die Drosselstellung geschaltet wird, wenn mindestens eine der folgenden Bedingungen erfüllt ist:
der Insufflationsdruck (p) beträgt mindestens für eine vorgegebene Dauer mindestens 80 %, vorzugsweise mindestens 90 % des Insufflationssolldrucks;
der Insufflationssolldruck ist mindestens für eine vorgegebene Dauer konstant;
die Motordrehzahl liegt mindestens für eine vorgegebene Dauer unterhalb eines Drehzahlschwellenwerts, der unter Berücksichtigung einer maximal möglichen Motordrehzahl so gewählt ist, dass ein beim Schalten der Drosseleinrichtung (23) in die Drosselstellung auftretender Abfall des Insufflationsdrucks (p) durch Erhöhen der Motordrehzahl über den Drehzahlschwellenwert hinaus gerade noch kompensiert werden kann.

4. Hustengerät (1) nach einem der vorhergehenden Ansprüche,
wobei das Insufflationsverfahren ferner umfasst:
Verhindern, dass die Drosseleinrichtung (23) in die Drosselstellung geschaltet wird, wenn mindestens eine der folgenden Bedingungen erfüllt ist:
der Insufflationssolldruck ist betragsmäßig größer als ein Exsufflationssolldruck, der bei einer Exsufflation am Patientenanschluss (17) anliegen soll;
der Insufflationssolldruck liegt über einem Druckschwellenwert, der unter Berücksichtigung eines maximal möglichen Insufflationsdrucks so gewählt ist, dass ein beim Schalten der Drosseleinrichtung (23) in die Drosselstellung auftretender Abfall des Insufflationsdrucks (p) in Höhe des Druckschwellenwerts durch Erhöhen der Motordrehzahl gerade noch kompensiert werden kann;
eine Funktion zum Bewirken einer gezielten Oszillation des Insufflationsdrucks (p) ist aktiviert.

5. Hustengerät (1) nach einem der vorhergehenden Ansprüche,
wobei die Drosseleinrichtung (23) in mindestens eine zusätzliche Drosselstellung schaltbar ist und ausgebildet ist, um den Verbindungskanal (15) beim Schalten in die zusätzliche Drosselstellung anders als beim Schalten in die Drosselstellung zu verengen, wobei die Drosseleinrichtung (23) beim Umschalten zwischen der Ausgangsstellung und der Drosselstellung die zusätzliche Drosselstellung passiert.

6. Hustengerät (1) nach Anspruch 5,
wobei die Drosseleinrichtung (23) beim Schalten in die Drosselstellung zunächst mit einer ersten Schaltgeschwindigkeit von der Ausgangsstellung in die zusätzliche Drosselstellung und anschließend mit einer zweiten Schaltgeschwindigkeit von der zusätzlichen Drosselstellung in die Drosselstellung bewegt wird.

7. Hustengerät (1) nach einem der vorhergehenden Ansprüche,
wobei die Steuereinrichtung (27) ausgebildet ist, um ferner folgendes Exsufflationsverfahren auszuführen:
Schalten der Koppeleinrichtung (21) in die Exsufflationsstellung;
Schalten der Drosseleinrichtung (23) in die Ausgangsstellung;
Empfangen des Drucksignals (29), wobei das Drucksignal (29) einen am Patientenanschluss (17) anliegenden Exsufflationsdruck (p) als den erfassten Druck (p) anzeigt;
Bestimmen einer Exsufflationsdruckabweichung zwischen dem Exsufflationsdruck (p) und einem Exsufflationssolldruck;
Steuern des Antriebsmotors (9), um die Exsufflationsdruckabweichung zu verringern.

8. Hustengerät (1) nach Anspruch 7,
wobei die Koppeleinrichtung (21) ferner in mindestens eine Zwischenstellung schaltbar ist und ausgebildet ist, um den Druckanschluss (13) in der Zwischenstellung sowohl mit der Ansaugöffnung (5) als auch mit der Ausblasöffnung (7) fluidisch zu koppeln;
wobei das Exsufflationsverfahren ferner umfasst:
Schalten der Koppeleinrichtung (21) von der Exsufflationsstellung in die Zwischenstellung, um unerwünschte Schwankungen des Exsufflationsdrucks (p) zu dämpfen.

9. Hustengerät (1) nach Anspruch 8,
wobei die Koppeleinrichtung (21) unter Berücksichtigung eines zeitlichen Verlaufs (33) des Exsufflationsdrucks (p) und/oder eines zeitlichen Verlaufs der Motordrehzahl in die Zwischenstellung geschaltet und/oder in der Zwischenstellung gehalten wird.

10. Hustengerät (1) nach einem der Ansprüche 7 bis 9,
wobei die Drosseleinrichtung (23) ferner zwischen einer ersten Oszillationsstellung und einer zweiten Oszillationsstellung umschaltbar ist und ausgebildet ist, um den Verbindungskanal (15) beim Schalten von der ersten Oszillationsstellung in die zweite Oszillationsstellung zu verengen;
wobei das Exsufflationsverfahren ferner umfasst:
Abwechselndes Umschalten der Drosseleinrichtung (23) zwischen der ersten Oszillationsstellung und der zweiten Oszillationsstellung, um eine gezielte Oszillation des Exsufflationsdrucks (p) zu bewirken.

11. Hustengerät (1) nach einem der vorhergehenden Ansprüche, ferner umfassend:
einen Flusssensor (39), der ausgebildet ist, um einen Volumenstrom (Q) durch den Verbindungskanal (15) zu erfassen und ein den erfassten Volumenstrom (Q) anzeigendes Flusssignal (37) zu erzeugen;
wobei die Steuereinrichtung (27) ausgebildet ist, um ferner das Flusssignal (37) zum Schalten der Koppeleinrichtung (21) und/oder der Drosseleinrichtung (23) und/oder zum Steuern des Antriebsmotors (9) auszuwerten.

12. Hustengerät (1) nach Anspruch 11 rückbezogen auf Anspruch 10,
wobei das Exsufflationsverfahren ferner umfasst:
Empfangen des Flusssignals (37), wobei das Flusssignal (37) einen exsufflierten Volumenstrom (Q) als den erfassten Volumenstrom (Q) anzeigt;
wobei das abwechselnde Umschalten zum Bewirken der gezielten Oszillation erst dann erfolgt, wenn der exsufflierte Volumenstrom (Q) ein lokales Maximum (PCF) erreicht.

13. Hustengerät (1) nach einem der vorhergehenden Ansprüche,
wobei die Drosseleinrichtung (23) ein um eine Drehachse (43) zwischen der Ausgangsstellung und der Drosselstellung drehbar gelagertes Stellglied (45) und einen elektrischen Aktor (31) zum Drehen des Stellglieds (45) umfasst, wobei das Stellglied (45) einen Wandabschnitt (47) zum Verringern eines Strömungsquerschnitts des Verbindungskanals (15) umfasst und so ausgebildet ist, dass der Wandabschnitt (47) in der Drosselstellung weiter in den Verbindungskanal (15) hineinragt als in der Ausgangsstellung.

14. Hustengerät (1) nach einem der vorhergehenden Ansprüche, ferner umfassend:
einen in den Verbindungskanal (15) mündenden Belüftungskanal (35) zum Ermöglichen eines Druckausgleichs mit einer Umgebung des Verbindungskanals (15);
wobei die Drosseleinrichtung (23) ausgebildet ist, um den Belüftungskanal (35) in der Ausgangsstellung zu verschließen und in der Drosselstellung freizugeben.

15. Beatmungsgerät (49) zum invasiven und/oder nicht invasiven Beatmen eines Patienten, wobei das Beatmungsgerät (49) ein Hustengerät (1) nach einem der vorhergehenden Ansprüche umfasst.

## Claims

1. A coughing device (1) for assisting a patient in coughing, wherein the coughing device (1) comprises:
a blower (3) comprising an inlet port (5), an outlet port (7), a drive motor (9), and a rotor (11) coupled to the drive motor (9) for conveying a gas from the inlet port (5) to the outlet port (7) as a function of the motor speed of the drive motor (9);
a pressure port (13);
a patient port (17) for connecting a patient interface;
a connecting channel (15) that fluidly connects the pressure port (13) to the patient port (17);
a coupling apparatus (21) that is switchable between an insufflation position and an exsufflation position and is designed to fluidly couple the pressure port (13) to the outlet port (7) in the insufflation position and to the inlet port (5) in the exsufflation position;
a throttling apparatus (23) that is switchable between an initial position and a throttled position and is designed to constrict the connecting channel (15) upon switching from the initial position to the throttled position;
a pressure sensor (25) designed to detect a pressure (p) prevailing at the patient port (17) and to generate a pressure signal (29) indicating the detected pressure (p);
a control apparatus (27) for controlling operation of the coughing device (1), wherein the control apparatus (27) is designed to perform the following insufflation method:
switching of the coupling apparatus (21) into the insufflation position;
receiving the pressure signal (29), wherein the pressure signal (29) indicates, as the detected pressure (p), an insufflation pressure (p) present at the patient port (17);
determining an insufflation pressure deviation between the insufflation pressure (p) and a target insufflation pressure;
controlling the drive motor (9) to reduce the insufflation pressure deviation;
**characterized in that** the insufflation method further comprises:
switching the throttling apparatus (23) from the initial position into the throttled position and holding the throttling apparatus (23) in the throttled position while controlling the drive motor (9) to effect an additional increase in the motor speed, wherein the thus increased motor speed is maintained at least until switching of the coupling apparatus (21) into the exsufflation position to enable provision of a correspondingly increased flow rate for increasing the peak cough flow in a subsequent exsufflation.

2. The coughing device (1) according to claim 1,
wherein the throttling apparatus (23) is held in the throttled position at least until the coupling apparatus (21) is switched to the exsufflation position; and/or
wherein the throttling apparatus (23), taking into account a time profile (33) of the insufflation pressure (p) and/or a time profile of the motor speed, is switched into the throttled position and/or is held in the throttled position.

3. The coughing device (1) according to one of the preceding claims,
wherein the throttling apparatus (23) is switched to the throttled position when at least one of the following conditions is met:
the insufflation pressure (p) is, for at least a predefined duration, at least 80%, preferably at least 90%, of the target insufflation pressure;
the target insufflation pressure is constant for at least a predefined duration;
the motor speed is below a speed threshold for at least a predefined duration, the speed threshold being selected, taking into account the maximum possible motor speed, such that a drop in the insufflation pressure (p) that occurs upon switching the throttling apparatus (23) into the throttled position can just barely be compensated for by increasing the motor speed beyond the speed threshold.

4. The coughing device (1) according to one of the preceding claims,
wherein the insufflation method further comprises:
preventing the throttling apparatus (23) from being switched to the throttled position when at least one of the following conditions is met:
the target insufflation pressure is, in terms of magnitude, greater than a target exsufflation pressure that is to be present at the patient port (17) during an exsufflation;
the target insufflation pressure is above a pressure threshold, which, taking into account a maximum possible insufflation pressure, is selected such that a drop in the insufflation pressure (p), in an amount equal to the pressure threshold, that occurs upon switching the throttling apparatus (23) into the throttled position can just barely be compensated for by increasing the motor speed;
a function for effecting a targeted oscillation of the insufflation pressure (p) is activated.

5. The coughing device (1) according to one of the preceding claims,
wherein the throttling apparatus (23) is switchable into at least one additional throttled position and is designed to constrict the connecting channel (15) upon switching into the additional throttled position differently than upon switching into the throttled position, wherein the throttling apparatus (23), when switching between the initial position and the throttled position, passes through the additional throttled position.

6. The coughing device (1) according to claim 5,
wherein, when switching into the throttled position, the throttling apparatus (23) is moved initially at a first switching speed from the initial position to the additional throttled position and subsequently at a second switching speed from the additional throttled position to the throttled position.

7. The coughing device (1) according to one of the preceding claims,
wherein the control apparatus (27) is designed to further perform the following exsufflation method:
switching the coupling apparatus (21) into the exsufflation position;
switching the throttling apparatus (23) into the initial position;
receiving the pressure signal (29), wherein the pressure signal (29) indicates, as the detected pressure (p), an exsufflation pressure (p) present at the patient port (17);
determining an exsufflation pressure deviation between the exsufflation pressure (p) and a target exsufflation pressure;
controlling the drive motor (9) to reduce the exsufflation pressure deviation.

8. The coughing device (1) according to claim 7,
wherein the coupling apparatus (21) is further switchable into at least one intermediate position and is designed, in the intermediate position, to fluidly couple the pressure port (13) to both the inlet port (5) and the outlet port (7);
wherein the exsufflation method further comprises:
switching the coupling apparatus (21) from the exsufflation position to the intermediate position in order to dampen undesired fluctuations in the exsufflation pressure (p).

9. The coughing device (1) according to claim 8,
wherein, taking into account a time profile (33) of the exsufflation pressure (p) and/or a time profile of the motor speed, the coupling apparatus (21) is switched into the intermediate position and/or held in the intermediate position.

10. The coughing device (1) according to one of claims 7 to 9,
wherein the throttling apparatus (23) is further switchable between a first oscillation position and a second oscillation position and is designed to narrow the connecting channel (15) upon switching from the first oscillation position to the second oscillation position;
wherein the exsufflation method further comprises:
alternately switching the throttling apparatus (23) between the first oscillation position and the second oscillation position in order to effect a targeted oscillation of the exsufflation pressure (p).

11. The coughing device (1) according to one of the preceding claims, further comprising:
a flow sensor (39) designed to detect a flow rate (Q) through the connecting channel (15) and to generate a flow signal (37) indicating the detected flow rate (Q);
wherein the control apparatus (27) is designed to further evaluate the flow signal (37) for switching the coupling apparatus (21) and/or the throttling apparatus (23) and/or for controlling the drive motor (9).

12. The coughing device (1) according to claim 11, as dependent on claim 10,
wherein the exsufflation method further comprises:
receiving the flow signal (37), wherein the flow signal (37) indicates an exsufflated flow rate (Q) as the detected flow rate (Q);
wherein the alternating switching for effecting the targeted oscillation occurs only when the exsufflated flow rate (Q) reaches a local maximum (PCF).

13. The coughing device (1) according to one of the preceding claims,
wherein the throttling apparatus (23) comprises an actuator member (45) mounted about an axis of rotation (43) so as to be rotatable between the initial position and the throttled position, and an electric actuator (31) for rotating the actuator member (45), wherein the actuator member (45) comprises a wall section (47) for reducing the flow cross-section of the connecting channel (15) and is designed such that the wall section (47) projects further into the connecting channel (15) in the throttled position than in the initial position.

14. The coughing device (1) according to one of the preceding claims, further comprising:
a vent channel (35) opening into the connecting channel (15) for enabling pressure equalization with an ambient environment of the connecting channel (15);
wherein the throttling apparatus (23) is designed to close the vent channel (35) in the initial position and to open it in the throttled position.

15. A ventilator (49) for invasive and/or non-invasive ventilation of a patient, wherein the ventilator (49) comprises a coughing device (1) according to one of the preceding claims.

## Revendications

1. Dispositif d'assistance à la toux (1) destiné à aider un patient à tousser, ledit dispositif d'assistance à la toux (1) comprenant :
une soufflante (3) comprenant un orifice d'aspiration (5), un orifice d'évacuation (7), un moteur d'entraînement (9) et un rotor (11) couplé au moteur d'entraînement (9) pour acheminer un gaz de l'orifice d'aspiration (5) vers l'orifice d'évacuation (7) en fonction de la vitesse du moteur d'entraînement (9) ;
un raccord de pression (13) ;
un raccord patient (17) destiné au raccordement d'une interface patient ;
un canal de liaison (15) reliant fluidiquement le raccord de pression (13) au raccord patient (17) ;
un dispositif de couplage (21), commutable entre une position d'insufflation et une position d'exsufflation et configuré pour coupler de manière fluidique le raccord de pression (13) à l'orifice d'évacuation (7) en position d'insufflation et à l'orifice d'aspiration (5) en position d'exsufflation ;
un dispositif d'étranglement (23), commutable entre une position initiale et une position d'étranglement et configuré pour rétrécir le canal de liaison (15) lors de la commutation de la position initiale vers la position d'étranglement;
un capteur de pression (25) configuré pour détecter la pression (p) présente au niveau du raccord patient (17) et pour générer un signal de pression (29) indiquant la pression (p) détectée ;
une unité de commande (27) destinée à commander un fonctionnement du dispositif d'assistance à la toux (1), ladite unité de commande (27) étant configurée pour mettre en œuvre le procédé d'insufflation suivant :
commutation du dispositif de couplage (21) en position d'insufflation ;
réception du signal de pression (29), ledit signal de pression (29) indiquant une pression d'insufflation (p) présente au niveau du raccord patient (17) comme pression (p) détectée ;
détermination d'un écart de pression d'insufflation entre la pression d'insufflation (p) et une pression d'insufflation de consigne ;
commande du moteur d'entraînement (9) afin de réduire l'écart de pression d'insufflation ;
**caractérisé en ce que** le procédé d'insufflation comprend en outre :
la commutation du dispositif d'étranglement (23) de la position initiale vers la position d'étranglement et le maintien du dispositif d'étranglement (23) en position d'étranglement lors de la commande du moteur d'entraînement (9), afin de provoquer une augmentation supplémentaire de la vitesse du moteur, la vitesse du moteur ainsi augmentée étant maintenue au moins jusqu'au moment de la commutation du dispositif de couplage (21) en position d'exsufflation, afin de permettre la mise à disposition d'un flux volumique augmenté en conséquence pour accroître le débit de pointe de toux lors d'une exsufflation ultérieure.

2. Dispositif d'assistance à la toux (1) selon la revendication 1,
dans lequel le dispositif d'étranglement (23) est maintenu en position d'étranglement au moins jusqu'à ce que le dispositif de couplage (21) soit commuté en position d'exsufflation ; et/ou
dans lequel le dispositif d'étranglement (23) est commuté en position d'étranglement et/ou maintenu en position d'étranglement en tenant compte d'une évolution temporelle (33) de la pression d'insufflation (p) et/ou d'une évolution temporelle de la vitesse du moteur.

3. Dispositif d'assistance à la toux (1) selon l'une des revendications précédentes,
dans lequel le dispositif d'étranglement (23) est commuté en position d'étranglement lorsqu'au moins l'une des conditions suivantes est remplie :
la pression d'insufflation (p) est égale, au moins pour une durée prédéfinie, à au moins 80 %, de préférence à au moins 90 %, de la pression d'insufflation de consigne ;
la pression d'insufflation de consigne est constante au moins pour une durée prédéfinie ;
la vitesse du moteur demeure, au moins pour une durée prédéfinie, inférieure à un seuil de vitesse choisi en tenant compte d'une vitesse maximale du moteur de manière à ce qu'une chute de la pression d'insufflation (p), survenant lors de la commutation du dispositif d'étranglement (23) en position d'étranglement, puisse être tout juste compensée par une augmentation de la vitesse du moteur au-delà du seuil de vitesse.

4. Dispositif d'assistance à la toux (1) selon l'une quelconque des revendications précédentes,
dans lequel le procédé d'insufflation comprend en outre :
le fait d'empêcher que le dispositif d'étranglement (23) ne soit commuté en position d'étranglement lorsqu'au moins l'une des conditions suivantes est remplie :
la pression d'insufflation de consigne est, en valeur absolue, supérieure à la pression d'exsufflation de consigne destinée à être appliquée au niveau du raccord patient (17) lors d'une exsufflation ;
la pression d'insufflation de consigne est supérieure à un seuil de pression choisi en tenant compte d'une pression maximale d'insufflation possible de manière à ce qu'une chute de la pression d'insufflation (p), d'une valeur égale audit seuil de pression, survenant lors de la commutation du dispositif d'étranglement (23) en position d'étranglement, puisse tout juste être compensée par augmentation de la vitesse du moteur;
une fonction destinée à produire une oscillation ciblée de la pression d'insufflation (p) est activée.

5. Dispositif d'assistance à la toux (1) selon l'une quelconque des revendications précédentes,
dans lequel le dispositif d'étranglement (23) est commutable en au moins une position d'étranglement supplémentaire et configuré pour rétrécir le canal de liaison (15) lors de la commutation vers la position d'étranglement supplémentaire d'une manière différente que lors de la commutation vers la position d'étranglement, le dispositif d'étranglement (23) passant par la position d'étranglement supplémentaire lors de la commutation entre la position initiale et la position d'étranglement.

6. Dispositif d'assistance à la toux (1) selon la revendication 5,
dans lequel le dispositif d'étranglement (23), lors de la commutation en position d'étranglement, est d'abord déplacé selon une première vitesse de commutation de la position initiale vers la position d'étranglement supplémentaire, puis selon une deuxième vitesse de commutation de la position d'étranglement supplémentaire vers la position d'étranglement.

7. Dispositif d'assistance à la toux (1) selon l'une quelconque des revendications précédentes,
dans lequel l'unité de commande (27) est configurée pour mettre en œuvre en outre le procédé d'exsufflation suivant :
commutation du dispositif de couplage (21) en position d'exsufflation ;
commutation du dispositif d'étranglement (23) en position initiale ;
réception du signal de pression (29), le signal de pression (29) indiquant, en tant que pression détectée (p), une pression d'exsufflation (p) présente au niveau du raccord patient (17) ;
détermination d'un écart de pression d'exsufflation entre la pression d'exsufflation (p) et une pression d'exsufflation de consigne ;
commande du moteur d'entraînement (9) afin de réduire l'écart de pression d'exsufflation.

8. Dispositif d'assistance à la toux (1) selon la revendication 7,
dans lequel le dispositif de couplage (21) est en outre commutable en au moins une position intermédiaire, et configuré, dans la position intermédiaire, pour coupler fluidiquement le raccord de pression (13) à la fois à l'orifice d'aspiration (5) et à l'orifice d'évacuation (7) ;
dans lequel le procédé d'exsufflation comprend en outre :
la commutation du dispositif de couplage (21) de la position d'exsufflation vers la position intermédiaire, afin d'atténuer des fluctuations indésirables de la pression d'exsufflation (p).

9. Dispositif d'assistance à la toux (1) selon la revendication 8,
dans lequel le dispositif de couplage (21) est commuté en position intermédiaire et/ou maintenu en position intermédiaire, en tenant compte d'une évolution temporelle (33) de la pression d'exsufflation (p) et/ou d'une évolution temporelle de la vitesse du moteur.

10. Dispositif d'assistance à la toux (1) selon l'une quelconque des revendications 7 à 9,
dans lequel le dispositif d'étranglement (23) est en outre commutable entre une première position d'oscillation et une deuxième position d'oscillation, et configuré pour rétrécir le canal de liaison (15) lors de la commutation de la première position d'oscillation vers la deuxième position d'oscillation ;
dans lequel le procédé d'exsufflation comprend en outre :
la commutation alternée du dispositif d'étranglement (23) entre la première position d'oscillation et la deuxième position d'oscillation, afin de produire une oscillation ciblée de la pression d'exsufflation (p).

11. Dispositif d'assistance à la toux (1) selon l'une quelconque des revendications précédentes, comprenant en outre :
un capteur de débit (39) configuré pour détecter un flux volumique (Q) à travers le canal de liaison (15) et pour générer un signal de débit (37) indiquant le flux volumique (Q) détecté ;
dans lequel l'unité de commande (27) est en outre configurée pour exploiter le signal de débit (37) afin de commuter le dispositif de couplage (21) et/ou le dispositif d'étranglement (23) et/ou de commander le moteur d'entraînement (9).

12. Dispositif d'assistance à la toux (1) selon la revendication 11 dépendant de la revendication 10,
dans lequel le procédé d'exsufflation comprend en outre :
la réception du signal de débit (37), ledit signal de débit (37) indiquant un flux volumique d'exsufflation (Q) en tant que flux volumique (Q) détecté ;
dans lequel la commutation alternée destinée à produire l'oscillation ciblée n'a lieu que lorsque le flux volumique d'exsufflation (Q) atteint un maximum local (PCF).

13. Dispositif d'assistance à la toux (1) selon l'une quelconque des revendications précédentes,
dans lequel le dispositif d'étranglement (23) comprend un organe d'actionnement (45) monté de manière rotative autour d'un axe de rotation (43) entre la position initiale et la position d'étranglement, ainsi qu'un actionneur électrique (31) destiné à faire tourner l'organe d'actionnement (45), l'organe d'actionnement (45) comprenant une section de paroi (47) destinée à réduire une section d'écoulement du canal de liaison (15) et étant configuré de telle façon que la section de paroi (47) fait saillie plus loin dans le canal de liaison (15) en position d'étranglement qu'en position initiale.

14. Dispositif d'assistance à la toux (1) selon l'une quelconque des revendications précédentes, comprenant en outre :
un canal d'aération (35) débouchant dans le canal de liaison (15), destiné à permettre un équilibrage de pression avec un environnement du canal de liaison (15) ;
dans lequel le dispositif d'étranglement (23) est configuré pour fermer le canal d'aération (35) en position initiale et pour l'ouvrir en position d'étranglement.

15. Ventilateur (49) destiné à la ventilation invasive et/ou non invasive d'un patient, le ventilateur (49) comprenant un dispositif d'assistance à la toux (1) selon l'une quelconque des revendications précédentes.
